# EUROPEAN PATENT APPLICATION

(11) **EP 4 606 347 A1**
(43) Date of publication of application: **27.08.2025**
(21) Application number: 25158646.7
(22) Date of filing: 18.02.2025
(51) Int. Cl.: A61C 8/00, A61C 1/08, A61B 17/16, A61B 90/00

(54) **DRILL FOR A DENTAL IMPLANT**

(30) Priority: 21.02.2024 IT 202400003688
(71) Applicant: BC Medical S.r.l., 37131 Verona (VR) (IT)
(72) Inventor: BRUSCO, Matteo, Verona VR (IT); CHIARAMONTE, Nicola, Verona VR (IT)
(74) Representative: Manfrin, Marta

(57) **Abstract**

The present disclosure relates to a drill (10) for a dental implant adapted to be used in combination with a template (200) for a dental implant, wherein the drill (10) includes a shaft (11) extended in a longitudinal direction between a distal end (11b) and a proximal end (11a), wherein said distal end (11b) is intended to penetrate the tissues of a patient and the proximal end (11a) is intended to be coupled to a driving instrument, and wherein starting from the distal end (11b), the drill (10) has an operative milling portion (12) intended to mill a patient's bone, a coupling bush (20) suitable for coupling with a sleeve (21, 22) fixed to said template (200), and a stop body (30) arranged at least partially around the coupling bush (20). Furthermore the stop body (30) acts as a stop for said sleeve (21, 22) to determine a final coupling position of the coupling bush (20) with the relevant sleeve (21, 22), wherein the stop body (30) is mounted in a movable manner with respect to the coupling bush (20), to allow adjustment of the stop body (30) along the coupling bush (20) in a longitudinal direction, and wherein said drill (10) comprises a threaded fastening element (13) to lock the stop body (30) with respect to said coupling bush (20). Furthermore, the stop body (30) has on an internal side facing the coupling bush (20) a protruding portion (32) or bulk portion, and the coupling bush (20) has a recess or cavity (23), where the protruding portion (32) or bulk portion of the stop body (30) is received in the recess or cavity (23) of the coupling bush (20).

## Description

This disclosure generally relates to a drill for a dental implant. In particular, it preferably concerns a drill apt to be used in combination with a template for a dental implant. This disclosure also relates to a kit of drills that includes a plurality of said drills.

More specifically, generally, each drill has a shaft that defines a longitudinal direction of the drill. The drill also includes, starting from a distal end of the shaft, a working portion for milling the patient's bone, a coupling bush suitable for coupling with a sleeve, fixed to the template, wherein the coupling of the bush with the respective sleeve locks the longitudinal axis of the drill in position and defines a non-exceedable working depth in the tissues of the working portion.

Generally, in the field of tools for dental implant procedures, kits of various types of drills are used, wherein all of the kits shared by the fact of including a very large number of drills.

More specifically, a kit of drills typically requires a very large number of drills in order to cover various combinations of possible diameters and lengths based on the operative sequence to obtain the hole (in fact, generally, a small drill is used first to create a small hole, and then progressively larger drills are used to widen the hole until the final hole is obtained, i.e., having a diameter suitable for the insertion of the chosen dental implant).

It follows that, in order to create a hole for a dental implant, it is necessary to use drills with shaft of various diameters, from very small diameters to larger diameters, and that are as close as possible to the diameter corresponding to one of the final hole, which is near the diameter of the implant, and furthermore, it is necessary to provide drills in which the working portion is positioned at different depths, thus with coupling bush at different heights.

There is therefore the problem for a surgeon that for each implant, it is needed to pre-select only a few drills from the complex kit. These are therefore very complex kits, with numerous pieces that are very expensive and often not fully used.

In order to reduce the number of drills related to the desired milling depth, the use of an adjustable stop body has been proposed, which can be mounted in a movable manner compared to the coupling bush, to allow an adjustment of the stop body along the coupling bush in the longitudinal direction. The adjustable stop body allows of adjusting the desired milling depth. More specifically, the stop body, disposed at least partially around the coupling bush, serves as a stop to determine the final coupling position of the coupling bush with the respective sleeve.

The drill also includes a threaded grub screw to lock the stop body with respect to the coupling bush, that is, along the coupling bush at a desired height.

The threaded grub screw is screwed into the stop body until it engages the cylindrical body of the drill, in order to fix the stop body at a desired height/depth of the drill.

However, the stop body or arrest body and the respective threaded grub screw have large dimensions, and therefore the use of an adjustable stop body is not always useful due to the large size. In other words, there is an obstruction of the stop body and the threaded grub screw that prevents the use of the cutter in reduced spaces.

The disadvantage is even more critical in the case of large-diameter drills, used, for example, for final milling during an implant procedure, because the available working space is minimal. It follows that the use of a stop body to reduce the number of drills in relation to the desired depth is of little use for drills with a diameter closer to the final hole diameter.

Therefore, the prior art has not satisfactorily solved the problem of reducing the number of drills in a known kit. This problem has not been solved, particularly concerning drills with diameters close to the final hole diameter.

A technical problem at the basis of present disclosure lies in providing a drill for a dental implant, and a kit of drills for a dental implant, capable of overcoming the drawbacks of the prior art and/or achieving further advantages.

This problem is solved by a drill for dental implants and a kit of drills according to their respective independent claims. Secondary features of the subject of the present disclosure are defined in the respective dependent claims.

At the basis of the present disclosure is the important recognition by the authors that drills with an adjustable stop body provided with a threaded grub screw according to the prior art present the aforementioned large size due to the large external diameter of the stop body required to allow a sufficiently firm screwing of the threaded grab screw inside it, thus stabilizing the stop body to the drill.

Based on said recognition, a drill for dental implants is provided according to the present disclosure, preferably apt to be used in combination with a dental implant template, and which includes a shaft extending along a longitudinal direction between a distal end and a proximal end, where the distal end is intended to penetrate the tissues of a patient and the proximal end is intended to be coupled to a driving tool, preferably configured to rotate the drill.

In the context of the present disclosure, the term 'template' refers to a structure, preferably made of transparent resin, used in implant procedures to make the placement of the dental implant as precise as possible. The template also includes a reference and guide sleeve for creating the hole and the drill is inserted into the sleeve.

Furthermore, starting from the distal end, the drill includes a working portion for milling the patient's bone, a coupling bush suitable for coupling with the sleeve fixed to the template, and a stop body disposed at least partially around the coupling bush, wherein the stop body serves as a stop for the sleeve to determine the final coupling position of the coupling bush with the respective sleeve. In other words, the coupling bush can be disposed along the drill or form a portion of the drill between the distal end and the proximal end.

Furthermore, the stop body is mounted in a movable manner with respect to the coupling bush, to allow an adjustment of the stop body along the coupling bush in the longitudinal direction. The stop body, in other words, is longitudinally translatable, that is, along the longitudinal direction, with respect to to the coupling bush and is fixed/fixable to the coupling bush to define the maximum drilling depth of the drill. In particular, the drill includes a fastening element, such as a threaded grub screw, to lock the stop body relative to the coupling bush, that is, along the coupling bush at a desired height.

According to the present disclosure, the drill is characterized in that the stop body has, on an inner side facing the coupling bush, a protruding portion or bulk portion, and the coupling bush has a recess or cavity. Furthermore, the protruding portion or bulk portion of the stop body is received in the recess or cavity of the coupling bush.

Furthermore, according to the present disclosure, the threaded fastening element is screwed or screwable into the protruding portion or bulk portion of the stop body. That is, the threaded fastening element and the protruding portion or bulk portion of the stop body are mutually configured so that the threaded fastening element can be screwed into the protruding portion or bulk portion of the stop body.

According to a preferred aspect of the present disclosure, the protruding portion or bulk portion of the stop body includes or defines or is provided with a threaded through hole (nut) configured or intended to receive the threaded grub screw or couple with the threaded element. In other words, the protruding portion or bulk portion of the stop body is equipped, at the through hole, with a thread configured to allow for the screwing or unscrewing of the threaded fastening element relative to the stop body. Said threaded through hole, thanks to the depth of the protruding portion or bulk portion, contains a sufficient number of threads to securely screw the threaded grub screw.

According to a preferred aspect, the threaded through hole extends substantially radially with respect to the stop body, particularly in the protruding portion or bulk portion. In other words, in use, the threaded through hole extends substantially along a direction transverse to the longitudinal direction. Said differently again the threaded through hole extends between the outer surface or outer side of the stop body and the inner side or inner surface of the stop body.

In other words, once again, the protruding portion or bulk portion provides sufficient space to screw the threaded fastening element, such as a threaded grub screw, due to its development in the radial direction from the outside towards the inside of the stop body. In this way, it is not necessary to create the space required for screwing the threaded fastening element by increasing the external diameter of the stop body, which would otherwise excessively exceed the maximum external diameter of the bur, making it bulky, as happens in the prior art. Said differently, again, the projecting portion of the stop body extends substantially in a direction transverse to the longitudinal direction. The space between the outer surface or outer side and the inner surface or inner side of the stop body at the protruding portion or bulk portion offers sufficient space, that is, a sufficient length of the threaded through hole, to allow the screwing of the fastening element without protruding much from the outer surface or outer side of the coupling bush.

Thanks to this solution, the stop body can have, for the same diameter or with smaller diameters compared to the prior art, a greater volume/mass in the protruding portion or bulk portion for the fastening of the threaded body.

In other words, in the drill according to the present invention, the stop body has a compact external diameter, and at the same time, the threaded fastening element can be screwed into a sufficient number of threads to securely fasten the stop body along the coupling bush, thanks to the presence of the protruding portion or bulk portion.

In other words, once again, this advantage allows for a reduction in the number of drills of the kit not only for small diameters but also, and especially, for drill diameters close to the final hole size, as the available space in the oral cavity for such diameters is very limited. It follows that a technical advantage of the individual drill extends to the entire kit.

Regarding the recess or cavity of the coupling bush, it can have any shape, the important thing is that it is a recessed empty space - relative to the outer surface or outer side of the coupling bush - to accommodate the bulk portion.

In other words, if, for example, the coupling bush is preferably configured substantially as a cylinder, the recess or cavity can be formed, for example, by longitudinally cutting the cylinder starting from a chord positioned along a base circumference of the cylinder.

According to a preferred aspect, if the coupling bush is configured substantially as a cylinder, the recess or cavity can be formed, for example, by longitudinally removing, that is, along the longitudinal development direction, a portion of the cylinder starting from a chord positioned along a base circumference of the cylinder.

Said differently again, for example, said recess or cavity on the cylindrical surface of the coupling bush is formed by longitudinally cutting a circular sector from the coupling bush itself.

According to a preferred aspect of the present disclosure, the coupling bush has an outer surface. In particular, according to this preferred aspect, the coupling bush includes, along the longitudinal direction, a distal part and a proximal part. The outer surface of the coupling bush at the distal part is substantially cylindrical, and the outer surface at said proximal part has a substantially semicylindrical portion coupled with a flat portion; and wherein the recess or cavity is defined at the flat portion or corresponds to the flat portion.

It follows that, for example, the protruding portion or bulk portion of the stop body has a complementary shape to the recess or cavity, that is, a shape that can occupy or fill the free space between the stop body and the coupling bush formed by the recess or cavity.

Preferably, the outer surface or outer side of the coupling bush and the inner surface or inner side of the stop body are not threaded, that is, they are without threads, more preferably they are substantially smooth. As mentioned earlier, the outer surface or outer side of the coupling bush and the inner side or inner surface of the stop body face each other. In other words, the coupling between the coupling bush and the stop body is not the typical coupling between two threaded surfaces.

Furthermore, the transversal section (that is, transversal to the longitudinal direction) of the coupling bush is reduced thanks to the presence of the cavity obtained, for example, in the manner described earlier. The space left free or empty by the cavity is therefore occupied or occupiable by the stop body, in particular by the protruding portion or bulk portion. According to a preferred aspect of the present disclosure, this protruding portion or bulk portion predominantly develops in the radial direction, that is, in a direction transverse to the longitudinal direction. In other words, the protruding portion or bulk portion predominantly develops towards the center of the stop body. Preferably, the protruding portion or bulk portion is configured such that it has sufficient material, in terms of, for example, thickness or depth and width, sufficient to create the threaded through hole. Preferably, the protruding portion or bulk portion has a thickness or depth sufficient to allow the creation, in correspondence with this protruding portion or bulk portion, of the threaded through hole.

It can be understood, from the geometry of the parts defined above, that the stop body is a ring or toroidal body, that is, a body having a shape substantially like a ring or torus, which defines an internal through hole. The surface or inner side of the stop body surrounds or delimits this internal through hole. The protruding portion or bulk portion includes the threaded through hole; therefore, the inner surface of the stop body is not continuous but is in fact interrupted by the threaded through hole.

The protruding portion or bulk portion of the stop body is therefore preferably in alignment with the recess or cavity, that is, the protruding portion or bulk portion of the stop body has a complementary shape to the recess or cavity.

Preferably, the fastening element is a grub screw. And even more preferably, the protruding portion or bulk portion that characterizes the stop body is such that it contains within its thickness a sufficient number of threads to allow the threaded grub screw to be screwed in securely, thus allowing the stop body to be fixed to the coupling bush. As a result, the drill according to the present invention can occupy a smaller space compared to drills according to the prior art, especially for drills with large diameters nearly equal to the diameter of the hole to be created, and it can preferably rotate at low speed, for example preferably about 50 revolutions per minute.

In other words, again, the protruding portion or bulk portion allows increasing the available space inside the stop body for the threaded fastening element, without having to increase the outer diameter of the stop body, while at the same time ensuring a secure fastening of the stop body to the coupling bush.

That is to say, through the present invention, it is possible to obtain a dental implant surgery drill with adequate stability during the milling process, while having a reduced size in the direction perpendicular to the axis of rotation of the drill.

Consequently, the advantage of the drill according to the present invention is to achieve improved milling stability while maintaining a reduced horizontal size, that is, perpendicular to the axis of rotation of the drill, compared to the prior art.

The stop body is preferably adjustable in height along the coupling bush of the drill. Through the present invention, it is also possible to achieve improved safety during milling due to the presence of the stop body adjustable in height along the coupling bush.

Consequently, as said, a reduced number of drills with an adjustable stop body (and therefore with variable depth) can be used even with drills having a diameter very close to the diameter of the hole to be obtained, and thus when the available space is limited.

More specifically, the present disclosure also relates to a kit of drills for a dental implant, where the kit includes a plurality of drills, each of which is a drill according to the present invention. That is, each drill features one or more of the characteristics described above. The drills in the kit of drills differ from each other mainly by the diameter of the operative milling portion. This characteristic is enabled by the use of drills characterized by the coupling between the stop body and the coupling bush as described above. In particular, the operative milling portion of a first drill in the plurality of drills has a different diameter than the operative milling portion of a second drill in the plurality of drills. It has been observed that, for example, four or five drills can be provided with diameters ranging from 3 mm to 5 mm with a tolerance of 0.1 mm.

Meaning that, the drills in the plurality of drills that make up the kit each have a different diameter for the operative milling portion compared to the others. Preferably, this plurality of drills is a first plurality of drills, and the kit further includes a second plurality of drills. The two pluralities of drills differ in the diameter of the operative portion, and the second plurality of drills has drills with a smaller diameter that require a reducing body, preferably ring-shaped, disposed in the sleeve of the guide.

Even more preferably, the coupling bush of each drill in the second plurality of drills is indirectly coupleable with the sleeve of the guide. In particular, the kit preferably includes at least one ring-shaped reducting body designed to be inserted into the sleeve to reduce the diameter of the sleeve and to accommodate the coupling bush of at least one drill from the second plurality of drills, thereby indirectly coupling it with the sleeve of the guide. The second plurality of drills also preferably includes four or five drills.

With a reduced number of small drills, and the corresponding reducing body, all the necessary combinations of drills are therefore covered. It is clear that the kit of drills according to the present invention allows to obtain a reduced number of drills for dental implant procedures. This advantage is made possible by using drills as described above and is particularly beneficial, especially for drilling diameters close to the final hole diameter.

Other advantages, features, and methods of use of the object of the present disclosure will become evident from the following detailed description of some preferred embodiments, given by way of example and not limitation.

It is, however, evident that each embodiment may present one or more of the advantages listed above; in any case, it is not required that each embodiment simultaneously presents all of the listed advantages.

Reference will be made to the figures of the accompanying drawings, in which:

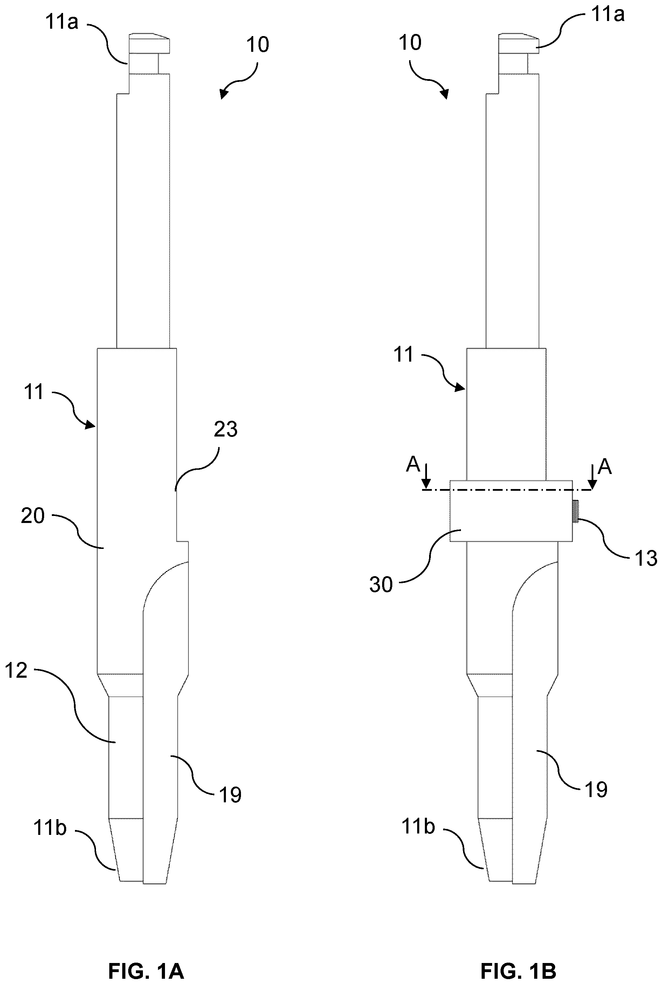

- Figure 1A shows a view of a drill from the first plurality of drills according to the present invention, without the stop body;
- Figure 1B shows the drill of Figure 1A associated with a stop body;

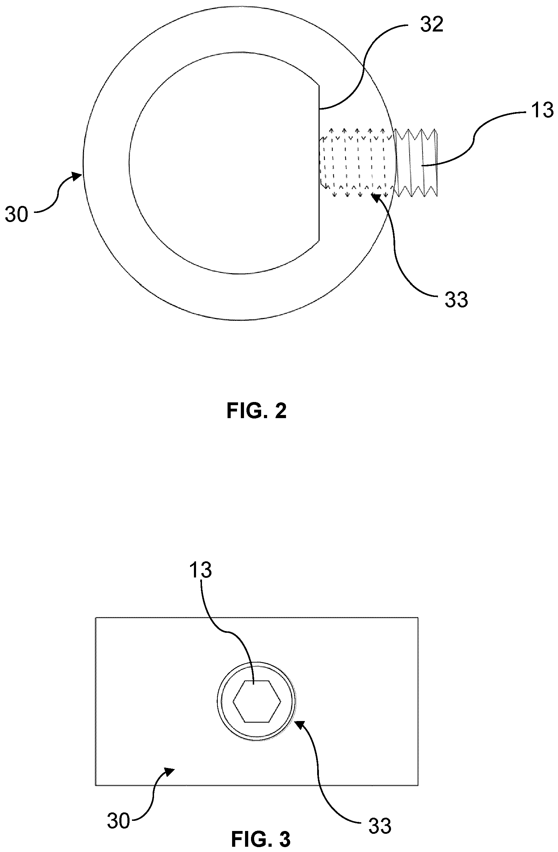

- Figure 2 shows a transverse sectional view along section A-A of Figure 1B;
- Figure 3 shows a lateral view of the stop body of Figure 1B;

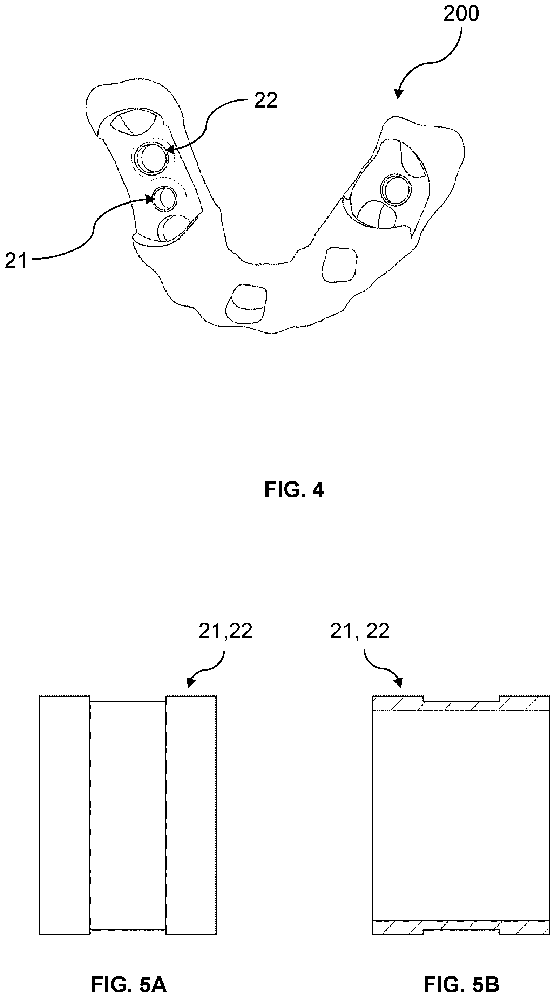

- Figure 4 shows a top view of a guide (template) that includes sleeves according to the present invention;
- Figure 5A shows a lateral view of one of the sleeves from Figure 4;
- Figure 5B shows a sectional view of the sleeve from Figure 5A;

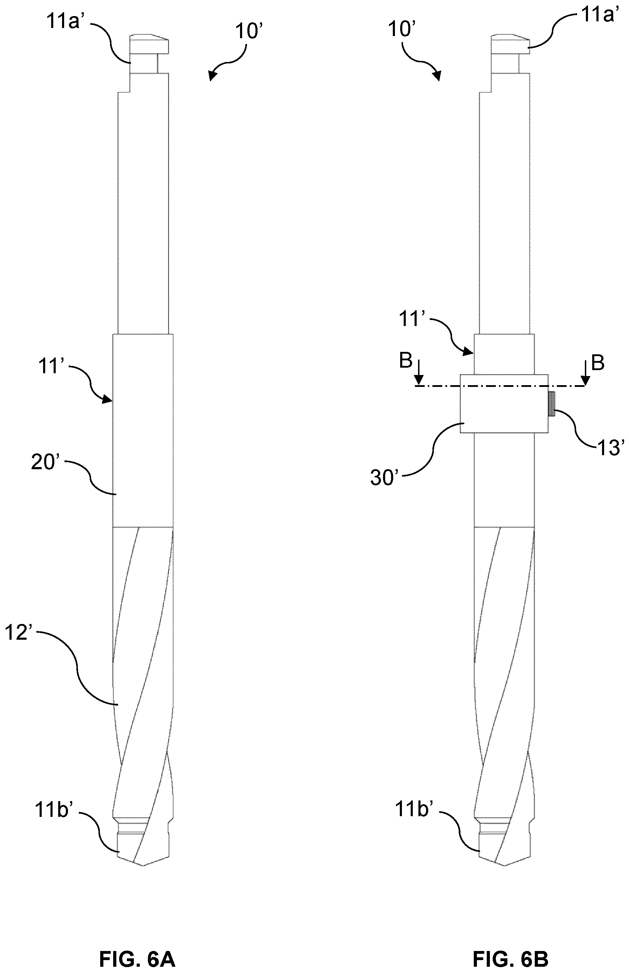

- Figure 6A shows a view of a drill from the second plurality of drills according to the present invention, without the stop body;
- Figure 6B shows the drill of Figure 6A associated with a stop body;

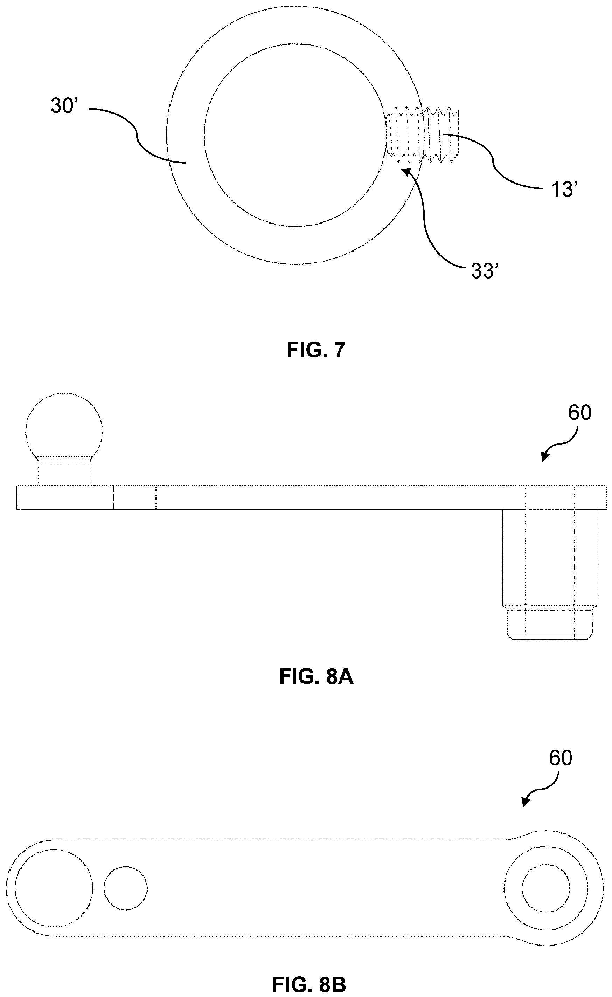

- Figure 7 shows a transverse sectional view along section B-B of Figure 6B;
- Figures 8A and 8B show, respectively, a lateral view and a top view of a ring reducing body according to the present invention;

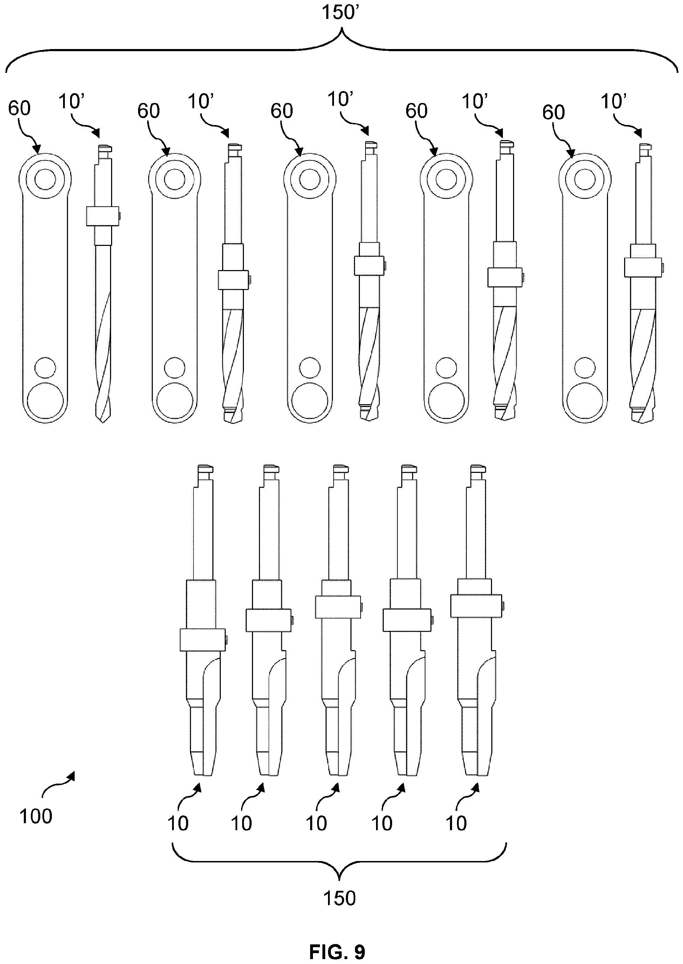

- Figure 9 shows a schematic view of a drill kit including a first plurality of drills, a second plurality of drills, and the respective ring reducing bodies according to the present invention.

With reference to the attached figures, the reference number 10 indicates a drill for a dental implant. The drill is preferably intended to be used in combination with a dental implant guide. The dental implant guide is preferably characterized by the presence of a stop sleeve positioned at the location wherein the hole is to be made.

In particular, the drill 10 includes a shaft 11 extending between a distal end 11b and a proximal end 11a, where the distal end 11b is intended to penetrate the bone of a patient, and the proximal end 11a is intended to be coupled with a driving tool, configured, for example, to rotate the drill 10 for performing the milling operation.

Additionally, starting from the distal end 11b, the drill 10 features an operational milling portion 12 intended for milling the bone of the patient, a coupling bush 20, suitable for coupling with a sleeve 21, 22 fixed to the template 200, and a stop body 30 arranged at least partially around the coupling bush 20.

In particular, the stop body 30 acts as a stop for the sleeve 21, 22 to determine the final coupling position of the coupling bush 20 with the respective sleeve 21, 22. The stop body 30 is mounted in a movable manner relative to the coupling bush 20, allowing for adjustment of the stop body 30 along the coupling bush 20 in the longitudinal direction. Additionally, the drill 10 includes a threaded fastening element 13 to secure the stop body 30 relative to the coupling bush 20.

Additionally, the drill according to the present invention is characterized by the fact that the stop body 30 has a protruding portion 32 or bulk portion on an inner side facing the coupling bush 20, and the coupling bush 20 has a recess or cavity 23. In particular, the protruding portion 32 or bulk portion of the stop body 30 is accommodated in the recess or cavity 23 of the coupling bush 20.

Said protruding portion 32 or bulk portion threaded through hole 33 extends along a direction perpendicular to the longitudinal direction of the drill. In particular, the threaded through hole 33 is intended and configured to receive the threaded fastening element 13.

In this way, the stop body 30 of the drill 10 is able to maintain a reduced external diameter compared to the prior art, since the threaded fastening element 13 also includes the protruding portion 32 or thickened portion to be screwed into the stop body, thus ensuring a secure fixation of the stop body 30 to the coupling bush 20 without resulting in an excessively bulky external diameter of the stop body 30.

Preferably, the protruding portion 32 or bulk portion of the stop body 30 is engaged in a shape-fitting manner within the recess or cavity 23 of the coupling bush 20. For example, the recess or cavity 23 may be formed by a cut along the longitudinal axis of the coupling bush 20, and the protruding portion 32 or bulk portion can occupy or be received within the recess or cavity 23.

Preferably, the protruding portion 32 or bulk portion of the stop body 30 has a convex shape with a curved surface, and the recess or cavity 23 of the coupling bush 20 has a corresponding concave shape with a curved surface. In particular, the recess or cavity 23 and the protruding portion 32 may have various shapes that are preferably complementary to each other.

The external diameter of the stop body 30, according to the present invention, slightly exceeds the internal diameter of the sleeve 21, 22, or the ring-shaped reducing body 60 with which it is to couple. From the geometry of the parts defined above, it can be understood that the stop body 30 is a ring or toroidal shaped body that defines an internal through hole. The protruding portion or bulk portion includes the threaded through hole 33; therefore, the internal surface of the stop body is not continuous but is in fact interrupted by the threaded through hole 33.

Furthermore, preferably, the coupling bush 20 and the stop body 30 have surfaces that are either free of threads or, more preferably, are substantially smooth. More preferably, the external surface or outer side of the coupling bush 20 and the external surface or inner side of the stop body 30 are substantially smooth or, even more preferably, free of threads.

Preferably, the stop body 30 is ring or toroidal shaped body, for example, with an external diameter between 5 mm and 6 mm, and the protruding portion 32 or bulk portion occupies a part of the circumference on the inner side of the ring or torus. Preferably, the threaded fastening element 13 is a screw, for example with a length between 1.5 mm and 2.5 mm.

Preferably, the operative milling portion 12 has a diameter between 3 and 5 mm, with a tolerance of 0.1 mm.

Preferably, the operative milling portion 12 has a groove 19 for the removal of material from the bone of a patient. That is, along the operative milling portion 12 of each milling tool 10, a groove 19 is cut to allow the removal of bone material during the milling process.

This disclosure also relates to a kit of drills 100 for a dental implant, wherein the kit 100 includes a plurality 150 of drills, where each drill being a drill 10 according to one of the embodiments described in this disclosure and where the operative milling portion 12 of a first drill 10 of the plurality 150 of drills has a diameter different from the diameter of an operative milling portion 12 of a second drill 10 of the plurality 150 of drills.

That is to say, preferably, each drills of the plurality 150 of drills of the kit has a diameter of the operative milling portion different from that of the other drills in the plurality 150 of drills of the kit.

In particular, this kit features a significantly reduced number of drills compared to the prior art, thanks to the use of drills 10 that include a stop element of the type stop body 30. This characteristic applies to all the drills in the kit of drills 100, and is particularly advantageous for drills with a diameter close to the final hole size.

Preferably, the diameter of the operative milling portion 12 of the plurality 150 of drills of the kit is between 3 and 5 mm. Preferably, the kit includes five drills, where the diameter of the operative milling portion 12 of the first drill is 3 mm, the diameter of the operative milling portion 12 of the second drill is 3.5 mm, the diameter of the operative milling portion 12 of the third drill is 4 mm, the diameter of the operative milling portion 12 of the fourth drill is 4.5 mm, and the diameter of the operative milling portion 12 of the fifth drill is 5 mm. Preferably, the diameter of the operative milling portion 12 of the fourth drill is substantially equal to the diameter of the final hole to be created in the patient's tissues.

Preferably, the plurality 150 of drills is a first plurality of drills, and the kit 100 of drills further includes a second plurality 150' of drills, in which each drill 10' of the second plurality 150' includes a shaft 11' extending between a distal end 11b' and a proximal end 11a', wherein the distal end 11b' is intended to penetrate the tissues of a patient, and the proximal end 11a' is intended to be coupled to a driving tool.

In particular, starting from the distal end 11b', preferably each drill 10' of the second plurality 150' of drills includes an operational milling portion 12' intended to mill a bone of the patient, and a coupling bush 20' suitable for coupling with a sleeve 21, 22 fixed to the surgical guide 200.

In particular, preferably, the diameter of the operative milling portion 12' of the second plurality 150' of drills is between 2 mm and 4.2 mm.

Even more preferably, the coupling of the coupling bush 20' of each milling tool 10' of the second plurality 150' of milling tools with the sleeve 21, 22 fixed to the surgical guide 200 is an indirect coupling, and the kit includes at least one ring-shaped reducing body 60 to be inserted into the sleeve 21, 22 to reduce the diameter of the sleeve 21, 22. In particular, preferably, the coupling bush 20' of each milling tool 10' of the second plurality 150' of milling tools is intended to cooperate with the ring shaped reducing body 60.

That is to say, the ring-shaped reducing body 60 enables the indirect coupling between the sleeve 21, 22 and the drill of the second plurality 150' of drills, ensuring that the drills of the second plurality 150' remain stable during the milling operations and do not oscillate excessively.

Preferably, each milling tool 10' of the second plurality 150' of drills includes a stop body 30' disposed at least partially around the coupling bush 20', where the stop body 30' serves as a stop for the sleeve 21, 22 to determine the final coupling position of the coupling bush 20' with the respective sleeve 21, 22, ensuring the maximum milling depth.

In particular, preferably, the stop body is mounted in a movable manner with respect to the coupling bush 20', allowing adjustment of the stop body 30' along the coupling bush 20' in the longitudinal direction, and the drill 10' includes a threaded fastening element 13' to secure the stop body relative to the coupling bush 20'.

Preferably, the kit further includes a guide drill configured to perform a preliminary incision on a patient's bone to guide the milling operation of the first and/or second plurality 150' of drills.

Preferably, the kit also includes the template 200 and at least one sleeve 21, 22.

Preferably, the sleeve 21, 22 is a first sleeve for drill having a cutting portion diameter smaller than or equal to 4 mm, and the kit further includes a second sleeve for drills with cutting portions greater than 4 mm. For example, the drill in the kit of drills 100 with a diameter smaller than or equal to 4 mm are preferably configured to be associated with the first sleeve, while the drills in the kit of drills 100 with a diameter greater than 4 mm are preferably configured to be associated with the second sleeve.

Therefore, through the kit according to the present invention, it is possible to optimize the number of components required for dental implant procedures, as it is sufficient to use the first plurality 150 of drills for the corresponding diameters and the second plurality 150' of drills along with the reducing body 60 for the corresponding diameters, where all the drills from the first plurality 150 and the second plurality 150' can be coupled with a sleeve 21, 22 of the template. It should be noted that since the drills of the second plurality 150' are smaller, the stop body can also be made using prior art and may not feature the bulk portion.

Preferably, the drills of the first plurality 150 are configured to rotate between 50 and 80 revolutions per minute, while preferably the milling tools 10' of the second plurality 150' are configured to rotate between 800 and 1000 revolutions per minute.

In accordance with the present invention, it is also possible to combine the functionality of the reducing body for smaller diameter drills, in order to avoid oscillations during milling, with the convenience of adjusting the milling height using the stop body.

Furthermore, through the present invention, it is possible to achieve excellent safety during vertical milling while maintaining a reduced horizontal footprint, i.e., perpendicular to the rotation axis of the drill, especially for milling diameters greater than or equal to 4 mm, thanks to the recess or cavity of the coupling bush and the protruding portion or bulk portion of the stop body according to the present invention.

Therefore, through the present invention, it is possible to limit the risk of interference of the drill during milling operations with the surrounding anatomical areas of the patient, such as teeth or prosthetics, which could displace the correct vertical milling rotation axis. The subject matter of the present disclosure has been described so far with reference to its embodiments. It should be understood that other embodiments may exist, all falling within the scope of the claims outlined below.

## Claims

1. Drill (10) for a dental implant adapted to be used in combination with a template (200) for a dental implant, wherein the drill (10) includes a shaft (11) extended in a longitudinal direction between a distal end (11b) and a proximal end (11a), wherein said distal end (11b) is intended to penetrate the tissues of a patient and the proximal end (11a) is intended to be coupled to a driving instrument, and wherein starting from said distal end (11b), the drill (10) has an operative milling portion (12) intended to mill a patient's bone, a coupling bush (20) suitable for coupling with a sleeve (21, 22) fixed to said template (200), and a stop body (30) arranged at least partially around said coupling bush (20), wherein said stop body (30) acts as a stop for said sleeve (21, 22) to determine a final coupling position of the coupling bush (20) with the relevant sleeve (21, 22), wherein the stop body (30) is mounted in a movable manner with respect to said coupling bush (20), to allow adjustment of the stop body (30) along said coupling bush (20) in a longitudinal direction, and wherein said drill (10) comprises a threaded fastening element (13) to lock the stop body (30) with respect to said coupling bush (20), **characterized in that** the stop body (30) has on an internal side facing towards said coupling bush (20) a protruding portion (32) or bulk portion, and said coupling bush (20) has a recess or cavity (23), wherein the protruding portion (32) or bulk portion of the stop body (30) is received in the recess or cavity (23) of the coupling bush (20); and wherein the threaded fastening element (13) is screwed or screwable into the protruding portion (32) or bulk portion of the stop body (30).

2. Drill (10) for a dental implant according to claim 1, wherein the protruding portion (32) or bulk portion comprises a threaded through hole (33) configured in such a way that the threaded fastening element (13) can be screwable or screwed into said threaded hole (33).

3. Drill (10) for a dental implant according to claims 1 or 2, wherein said coupling bush (20) is substantially shaped like a cylinder, and wherein said recess or cavity (23) is obtained by longitudinally cutting the cylinder starting from a chord positioned along a base circumference of the cylinder.

4. Drill (10) for a dental implant according to any one of the preceding claims, wherein said coupling bush (20) has an external surface and wherein said external surface of said coupling bush (20) and the internal side of said stop body (30) are free of threads and/or are substantially smooth.

5. Drill (10) for a dental implant according to the preceding claim, wherein the protruding portion (32) or bulk portion of the stop body (30) is received with a form fit in the recess or cavity (23) of the coupling bush (20).

6. Drill (10) for a dental implant according to any of the preceding claims, wherein the protruding portion (32) or bulk portion of the stop body (30) has a convex shape with a curved surface, and the recess or cavity (23) of the coupling bush (20) has a corresponding concave shape with a curved surface.

7. Drill (10) for a dental implant according to any of the preceding claims, wherein the stop body (30) has the shape of a ring or torus, and said protruding portion (32) or bulk portion occupies a part of the circumference on the inner side of the ring or torus.

8. Drill (10) for a dental implant according to any of the preceding claims, wherein the threaded fastening element (13) is a grub screw.

9. Drill (10) for a dental implant according to any of the preceding claims, wherein the operative milling portion (12) has a diameter of between 3 and 5 mm.

10. Drill (10) for a dental implant according to any one of the preceding claims, wherein the operative milling portion (12) has a groove (19) for removing material to be excised from the tissues of a patient.

11. Kit of drills (100) for a dental implant wherein the kit (100) includes a plurality (150) of drills, wherein each drill is a drill (10) according to any one of the preceding claims, and wherein the operative milling portion (12) of a first drill (10) of the plurality of drills (150) has a different diameter from a diameter of an operative milling portion (12) of a second drill (10) of the plurality of drills (150).

12. Kit of drills (100) according to the preceding claim, wherein the diameter of the operative milling portion (12) of the plurality (150) of drills is between 3 and 5 mm.

13. Kit of drills (100) according to claim from 11 to 12, including five drills, and wherein the diameter of the operative milling portion (12) of the first drill is 3mm, the diameter of the operative milling portion (12) of the second drill is 3.5 mm, the diameter of the operative milling portion (12) of the third drill is 4 mm, the diameter of the operative milling portion (12) of the fourth drill is 4.5 mm, the diameter of the operative milling portion (12) of the fifth drill is 5 mm.

14. Kit of drills (100) according to any of the preceding claims 11 to 13, wherein said plurality (150) of drills is a first plurality of drills, and said kit of drills (100) further comprises a second plurality (150') of drills, wherein each drill (10') of said second plurality (150') of drills includes a shaft (11') extending between a distal end (11b') and a proximal end (11a'), wherein said distal end (11b') is intended to penetrate the tissues of a patient and the proximal end (11a') is intended to be coupled to a driving instrument, and wherein starting from said distal end (11b') each drill (10') of said second plurality (150') of drills has an operative milling portion (12') intended to mill a patient's bone, and a coupling bush (20') suitable for coupling with a sleeve (21, 22) fixed to said template (200), and wherein a diameter of an operative milling portion (12') of the second plurality (150') of drills is between 2 mm and 4.2 mm.

15. Kit of drills (100) according to the preceding claim wherein the coupling of the coupling bush (20') of each drill (10') of said second plurality (150') of drills with the sleeve (21, 22) fixed to said template (200) is an indirect coupling and said kit includes at least a ring-shaped reducing body (60) suitable for being inserted into said sleeve (21, 22) to reduce the diameter of the sleeve (21, 22), and wherein a coupling bush (20') of each drill (10') of the second plurality (150') of drills is adapted to cooperate with said ring-shaped reducing body (60).

16. Kit of drills (100) according to claim 14 or 15, wherein each drill (10') of said second plurality (150') of drills comprises a stop body (30') arranged at least partially around said coupling bush (20'), wherein said stop body (30') acts as a stop for said sleeve (21, 22) to determine a final coupling position of the coupling bush (20') with the relevant sleeve (21, 22), wherein the stop body is mounted in a movable manner with respect to said coupling bush (20'), to allow adjustment of the stop body (30') along said coupling bush (20') in a longitudinal direction, and wherein said drill (10') includes a threaded fastening element (13') to lock the stop body with respect to said coupling bush (20').

17. Kit of drills (100) according to any one of the preceding claims from 11 to 16, further comprising a guide drill configured to make a preliminary incision on a patient's bone to guide a milling by said plurality (150) of drills.

18. Kit of drills (100) according to any of the preceding claims from 11 to 17, including said template (200) and said sleeve (21, 22).

19. Kit of drills (100) according to the preceding claim, wherein said sleeve (21) is a first sleeve for drills having an operative milling portion between 0 and 4 mm and the kit of drills (100) includes a second sleeve for drills having operative milling portions greater than 4mm.
